# EUROPEAN PATENT APPLICATION

(11) **EP 4 036 214 A1**
(43) Date of publication of application: **03.08.2022**
(21) Application number: 22153790.5
(22) Date of filing: 27.01.2022
(51) Int. Cl.: C12N 1/00, A61K 35/744

(54) **NOVEL LACTOCOCCUS LACTIS STRAINS, COMPOSITIONS COMPRISING THEM, AND USE THEREOF IN PREVENTION AND TREATMENT OF INTESTINAL CANCER**

(30) Priority: 01.02.2021 PL 43682121
(71) Applicant: Instytut Biochemii I Biofizyki Polskiej Akademii Nauk, 02-106 Warszawa (PL)
(72) Inventor: SALANSKI, Przemyslaw, 07-230 Debinki (PL); BARDOWSKI, Jacek, 01-361 Warszawa (PL); SZCZEPANKOWSKA, Agnieszka, 05-520 Bielawa (PL); BOGUSLAWSKA, Joanna, 05-091 Zabki (PL)
(74) Representative: Grzelak, Anna

(57) **Abstract**

The object of the invention are novel bacterial strains *Lactococcus lactis* IBB109, deposited as B/00311, and *Lactococcus lactis* IBB417, deposited as B/00312, a composition, a dietary supplement, a probiotic preparation, a bacterial preparation comprising them, and uses thereof, particularly in the prevention and/or treatment of an intestinal cancer, preferably a colorectal cancer.

## Description

### FIELD OF THE INVENTION

The object of the invention are novel bacterial strains strains of bacteria: *Lactococcus lactis* IBB109, deposited as B/00311, and *Lactococcus lactis* IBB417, deposited as B/00312, with probiotic properties, a composition, a dietary supplement, a probiotic preparation, a bacterial preparation comprising them, and uses thereof, particularly in the prevention and/or treatment of intestinal cancer, preferably colorectal cancer.

### STATE OF THE ART

Lactic acid bacteria (LAB) are a diverse group of microorganisms used in the food industry, particularly in the dairy industry, but also in pharmacy. Among this group of bacteria, some strains possess probiotic properties. Studies have repeatedly confirmed the beneficial effect of LAB strains with probiotic properties on human or animal organism. Research results on the attempts to use LAB in anti-cancer therapy have been described (Liu et al. 2011, Banna et al. 2017).

Colorectal cancer (CRC) is one of the most common malignancies. In the world, one in three cases of cancer in men and one in two in women is a cancer of this type. Statistical data shows that in 2011 almost 6,000 men and 5,000 women in Poland died as a result of this disease. It is estimated that in 2025 the number of deaths may increase 2-3 times. Late-detected CRC is characterized by high mortality. It is estimated that approximately 1/3 of deaths due to neoplastic diseases are related to bad eating habits or lifestyle, e.g., low consumption of fruit and vegetables, lack of physical activity, and the use of tobacco and alcohol (Brenner et al. 2014). The above-mentioned factors are associated with dysbiosis of the normal intestinal microbiota. Disturbances in the composition of microorganisms living in the intestine lead, as a consequence, to the expansion of pathogenic bacteria, which due to their activity, e.g., the production of toxins, evoke inflammatory processes and carcinogenesis. According to experimental data, restoring the balance of the intestinal microbiota is often successfully supported by LAB strains (Skrzydlo-Radomanska and Wronecki 2018).

Microorganisms belonging to the LAB group are widely used in the food industry as starter strains in the production of fermented milk products, i.e. cheese, yoghurt, buttermilk. Research shows that in countries with high consumption of fermented food products (silages, dairy products) containing LAB, there is a lower incidence of CRC (Górska et al. 2019). The beneficial effect of LAB on human and animal health is historically documented, and has contributed to granting them the status of GRAS (generally recognized as safe) bacteria (Wasilewska et al. 2013, Collado et al. 2009).

Some LAB strains have probiotic properties that have a beneficial effect on the host organism, i.a. by producing substances that inhibit the growth of pathogenic bacteria, competing with them for nutrients and ecological niches, inhibiting the action of toxic substances, as well as stimulating the immune system of the host (Markowiak et al. 2017). The results of experimental studies indicate a beneficial effect of selected strains of LAB on the course and alleviation of symptoms of certain disease states, such as diabetes, obesity or chronic intestinal inflammation (Markowiak et al. 2017).

It is also suggested that LAB may be used as anti-cancer factors in CRC therapy (Mego et al. 2013). The rationale for this is provided by *in vitro* tests using model human colorectal cancer cell lines and in *in vivo* experiments on model animals, showing the ability of LAB strains to inhibit the growth of cancer cells or improve the immune response, by induction, among others, of proinflammatory and regulatory cytokines (Zhong et al. 2014, Jacouton et al. 2017, Górska et al. 2019). The anti-proliferative or pro-apoptotic activity of LAB in relation to colorectal cancer cells is a strain-dependent feature, and has been confirmed for selected strains, i.a. from the genus *Lactobacillus, Bifidobacterium* or *Pediococcus* (Yu and Li 2016). However, so far a similar effect has not been observed in LAB of the *Lactococcus* genus. The effect of probiotic preparations comprising lactic acid bacteria was also demonstrated in the treatment and prevention of disease development in patients with CRC (Yu and Li 2016). Other studies have shown that the consumption of kefir, containing 6 LAB strains, increases the cytotoxicity of NK cells of the KHYG-1 line against HCT116 colorectal cancer cells (Yamane et al. 2018). This effect was visible both when using live strains, as well as after their thermal inactivation.

The results of the experiments conducted so far show that carcinogenesis affects not only the cells of the immune system, but is also associated with disturbances in the expression of non-coding microRNA (miRNA) molecules (Deepak et al. 2016). Hence, it is believed that miRNAs produced by eukaryotic cells may be suitable biomarkers for the diagnosis, prognosis and prediction of cancer treatment outcomes. There are examples describing the effect of miRNA on the expression of key genes involved in proliferation, invasion and apoptosis, directly contributing to the carcinogenesis process in the colon (Wu et al. 2011). Studies suggest that the anti-cancer effect of probiotic preparations containing strains of lactic acid bacteria may be related to their influence on the expression of miRNA molecules, directly related to the regulation of genes involved in carcinogenesis processes (Kreuzer-Redmer et al. 2016, Heydari et al. 2019). It has been shown that unique strains of lactic acid bacteria, *Lactobacillus acidophilus* and *Bifidobacterium bifidum,* increase the expression of colorectal cancer suppressing miRNAs (Heydari et al. 2019). Strains of *Lactobacillus* sp. and *Bifidobacterium* sp., which affect the regulation of the levels of miR-122a and miR-146a molecules, which have an effect on the expression of the gene for occludin (a protein that seals the junctions of intestinal epithelial cells) and the TRAF6 and GSK-3β genes (involved in the innate immune response and inflammation) have also been described (Zhao et al. 2015, Zhou et al. 2015). Research shows that the effect of lactic acid bacteria on cancer cells is complex and specific only for certain strains.

Allowing the widespread use of LAB with an unknown genome sequence may lead to the increased colonization by opportunistic LAB which may evoke diseases, particularly in immunocompromised patients. Therefore, it is important to determine the biosafety of strains both at the genetic and physiological level.

The solutions known from the state of the art concern the group of LAB, which has a very high level of diversity. The influence of LAB on CRC is a strain-dependent property, therefore not all bacteria of the LAB group have health-promoting properties, and it is impossible to assume the overall influence of all LAB strains on the possible course of disease therapy. For this reason, unique LAB strains showing a therapeutic effect are still sought. The search is focused on strains showing a stronger, more stable and disease-specific therapeutic effect than the strains known from the state of the art.

### SUMMARY OF THE INVENTION

There is a continuous need to provide novel, unique, stable and safe LAB strains, which, due to their strain-dependent physiological properties, will act in the digestive tract in a specific way, by preventing development, limiting development and/or will be used in the therapy of intestinal cancer, and particularly, they will show a therapeutic effect on colorectal cancer cells. The broad spectrum of the inhibitory effect on cancer cells, with a simultaneous lack of such an effect on healthy cells is an important aspect in the potential use of bacterial strains in the therapy/prevention of cancer.

Unexpectedly, it was found that the novel strains of lactic acid bacteria: *Lactococcus lactis* IBB109 (deposited as **B/00311**) and the strain *Lactococcus lactis* IBB417 (deposited as **B/00312**), according to the invention, meet such expectations, they inhibit the growth and development of colorectal cancer cells, and their presence in the digestive tract will largely prevent and limit the development of intestinal cancers, especially colorectal cancer.

The present invention therefore provides novel strains of lactic acid bacteria: *L. lactis* IBB109 and *L. lactis* IBB417, with special probiotic and anti-cancer properties, especially against colorectal cancer cells. The strains were deposited on September 24, 2020 in the Polish Collection of Microorganisms (PCM) in Wroclaw under deposit numbers: **B/00311** - *Lactococcus lactis* IBB109 and **B/00312** - *Lactococcus lactis* IBB417.

The present invention also provides/specifies a pharmaceutical composition comprising the above-mentioned strains for use in the prevention and treatment of intestinal cancers, particularly colorectal cancer.

### DISCLOSURE OF THE INVENTION

In view of the described state of the art, the aim of the present invention is to overcome the indicated disadvantages and to provide novel, unique strains of *Lactococcus lactis,* **B/00311** and **B/00312,** and a pharmaceutical composition for use in the prevention and treatment of intestinal cancers, particularly colorectal cancer.

This aim was achieved through the analysis of several hundred strains of lactic acid bacteria isolated from their natural habitats, of which, among more than 50 tested bacterial strains of the *Lactococcus lactis* species, derived from natural sources, only the two strains according to the invention have properties that significantly inhibit the proliferation of colorectal adenocarcinoma cells, i.e. Caco-2 and HT-29 (ATCC collection - ATCC no. HTB-37 and ATCC no. HTB-38, respectively) *in vitro,* and at the same time they do not affect the development of healthy epithelial cells, kidney epithelial cells (HEK-293; ATTC no. CRL-1573), which allows their use in the prevention and treatment of intestinal cancer, particularly colorectal cancer.

It should be emphasized that the *Lactococcus lactis* IBB109 (B/00311) strain and the *Lactococcus lactis* IBB417 (B/00312) strain according to the invention have been tested for biosafety, and their genomes have been fully sequenced. It was found that they demonstrate the ability to survive in conditions of low pH and in the presence of bile salts at the level consistent with the available literature (Olszewska et al. 2013, Takanashi et al. 2014, Jatmiko et al. 2017), that is, they have features essential for successful passage through the upper digestive tract and longer survival in the digestive tract. Moreover, the IBB417 strain is characterized by strong adhesion to Caco-2 cells, what strengthens its potential to stay longer in the digestive tract.

The properties of *Lactococcus lactis* IBB109 (B/00311) and *Lactococcus lactis* IBB417 (B/00312) influence the inhibition of colorectal cancer cell development (*in vitro*); it is expected that the presence of the strains according to the invention in the digestive tract (*in vivo*) will greatly contribute to the prevention and reduction of the development of intestinal cancers, particularly colorectal cancer. The strains according to the invention significantly inhibit the proliferation of colorectal adenocarcinoma cells *in vitro* (as demonstrated on the Caco-2 and HT-29 cell lines), and at the same time do not affect and do not interfere with the normal development of healthy non-cancerous cells (as demonstrated on renal epithelial cells HEK-293).

Unexpectedly, it was found that the novel strains of *Lactococcus lactis* IBB109 (B/00311) and *Lactococcus lactis* IBB417 (B/00312) not only inhibit the growth of intestinal cancer cells, in particular colorectal adenocarcinoma cells, but also affect the expression of non-coding regulatory RNA molecules (microRNAs) associated with the proliferation of cancer cells (so-called oncomirs).

Intestinal cancer cells have a low level or show a lack of interleukin 18 (IL-18) expression, making them difficult to be recognized by the immune system of the host. Unexpectedly, it was found that the novel strains *of L. lactis* IBB109 (B/00311) and *L. lactis* IBB417 (B/00312) exert an immunomodulatory effect by inducing the secretion of the cytokine IL-18 in the cells of the colorectal adenocarcinoma line Caco-2.

At the same time, *L. lactis* IBB109 (B/00311) and *L. lactis* IBB417 (B/00312) show full biosafety in terms of the absence of antibiotic resistance genes in their genomes and the lack of hemolytic abilities.

The invention concerns a novel bacterial strain, *Lactococcus lactis* IBB109, deposited in the PCM under deposit no. B/00311.

The invention concerns a novel bacterial strain , *Lactococcus lactis* IBB417, deposited in the PCM under deposit no. B/00312.

The invention concerns a composition comprising novel bacterial strains - *Lactococcus lactis* IBB109, deposited in the PCM under deposit no. B/00311, and *Lactococcus lactis* IBB417, deposited in the PCM under deposit no. B/00312.

A preferred composition is a pharmaceutical composition further comprising a pharmaceutically acceptable carrier, more preferably the composition is in a liquid, solid form, e.g., powder, tablet, capsule for oral administration, etc.

The invention also concerns a pharmaceutical composition comprising the bacterial strain *Lactococcus lactis* IBB 109 according to the invention and/or the bacterial strain *Lactococcus lactis* IBB417 according to the invention, and/or a composition according to the invention, and a pharmaceutically acceptable carrier, for use as a medicament, preferably for use as a medicament in the prevention and/or treatment of an intestinal cancer, preferably in the prevention and/or treatment of a colorectal cancer.

Preferably the pharmaceutical composition is in liquid, solid form, e.g., powder, tablet, capsule for oral administration, etc.

The invention also concerns a dietary supplement comprising a unique bacterial strain *Lactococcus lactis* IBB109 according to the invention and/or a unique bacterial strain *Lactococcus lactis* IBB417 according to the invention and/or a composition according to the invention, wherein the dietary supplement is preferably in liquid, solid form, e.g., powder, tablet, capsule intended for oral administration, etc.

The invention also concerns a bacterial preparation containing the bacterial strain *Lactococcus lactis* IBB109 according to the invention and/or the bacterial strain *Lactococcus lactis* IBB417 according to the invention and/or a composition according to the invention for use as an active ingredient in a therapeutic preparation, functional food, dietary supplement, for use as an active ingredient of a medicament intended for the prevention and/or treatment of an intestinal cancer, preferably a colorectal cancer.

The invention also concerns the use of the bacterial strain *Lactococcus lactis* IBB 109 according to the invention and/or the bacterial strain *Lactococcus lactis* IBB417 according to the invention and/or the composition according to the invention as a functional additive in functional food, a functional drink additive, as an active ingredient in a probiotic preparation, as an active ingredient in a dietary supplement, as an active ingredient in a pharmaceutical composition.

The invention also concerns the use of the bacterial strain *Lactococcus lactis* IBB 109 according to the invention and/or the bacterial strain *Lactococcus lactis* IBB417 according to the invention and/or the composition according to the invention to induce a level of the cytokine IL-18 in intestinal cancer cells, preferably in colorectal cancer cells.

In the preferred embodiments according to the invention, the preparations containing the bacterial strain *Lactococcus lactis* IBB 109 according to the invention and/or the bacterial strain *Lactococcus lactis* IBB417 and/or the composition according to the invention comprise at least 10⁴ colony forming units (CFU)/mL, preferably 10⁷ CFU/mL, even more preferably 10⁸ CFU/mL.

In the preferred embodiments according to the invention, it is used in methods of therapy and prevention of colorectal cancer.

Preparations containing the bacterial strain *Lactococcus lactis* IBB 109 according to the invention and/or the bacterial strain *Lactococcus lactis* IBB417 according to the invention, and/or a composition containing them according to the invention, selectively inhibit the proliferation of colorectal cancer cells, while they do not adversely affect the proliferation of healthy (non-cancerous) cells.

Preparations containing the bacterial strain *Lactococcus lactis* IBB109 according to the invention and/or the bacterial strain *Lactococcus lactis* IBB417 according to the invention, and/or a composition containing them according to the invention, reduce the levels of microRNA molecules (oncomirs) in the colorectal cancer microenvironment, particularly such molecules as: let-7a, miR-21-5p, miR-221-3p, miR-18a-5p, miR-31-5p, miR-16-5, and possess immunomodulatory properties, increasing the level of IL-18, which is important in the regulation of the immune system response.

Both bacterial strains, *L. lactis* IBB109 and *L. lactis* IBB417, according to the invention are sensitive to ampicillin, vancomycin, gentamycin, kanamycin, streptomycin, erythromycin, clindamycin, tetramycin, chloramphenicol, and do not have hemolytic properties - therefore, they show biological safety when they are used enterally.

Both bacterial strains, *L. lactis* IBB109 and *L. lactis* IBB417, are resistant to low pH (pH 4), which increases their ability to survive in the upper digestive tract and to reach their target site (large intestine), which is not a feature of all bacteria of the genus *Lactococcus,* or lactic acid bacteria in general.

The bacterial strain *L. lactis* IBB109 shows good resistance to 0.1% (w/w) of bile salts, which increases its ability to survive in the upper digestive tract and reach the target site (large intestine). This is not a feature of all bacteria of the genus *Lactococcus,* or lactic acid bacteria in general.

The bacterial strain *L. lactis* IBB417 has a very good adhesive properties to Caco-2 cells and shows good adherence to mucins, which is a special feature and is not present in all bacteria of the genus *Lactococcus,* or lactic acid bacteria in general.

Therefore, in the state of the art, there are no known solutions regarding preparations based on *L. lactis* bacteria with such a documented *in vitro* influence on intestinal cancer cells, particularly on colorectal adenocarcinoma cells, therefore the solutions according to the invention constitute a significant progress and fulfill the significant need to provide novel natural and safe methods for the prevention and treatment of cancers, especially cancers of the intestine, including the large intestine.

### DESCRIPTION OF THE FIGURES

Exemplary embodiments of the invention are illustrated on the following figures, on which:

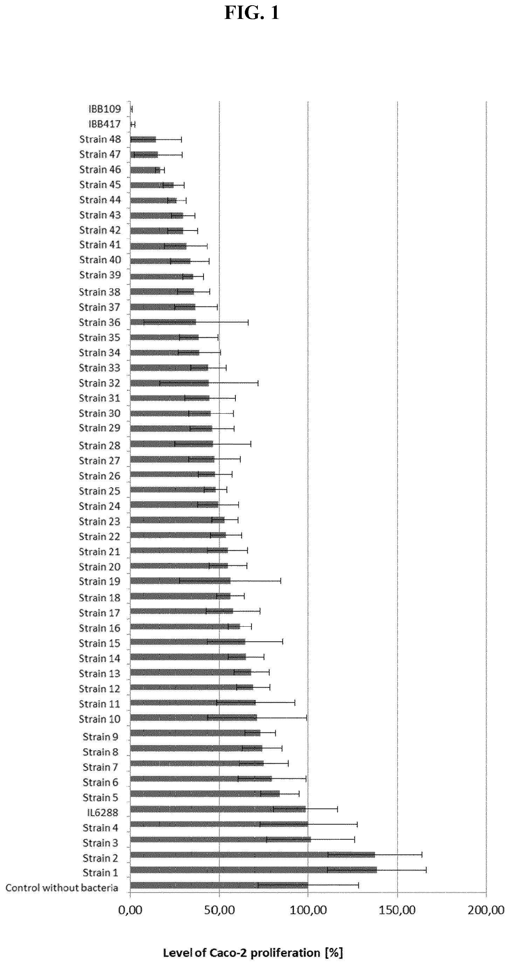

**Fig. 1**. shows the results of a screening experiment for the proliferation of Caco-2 cells in the presence of bacteria of the genus *Lactococcus.*

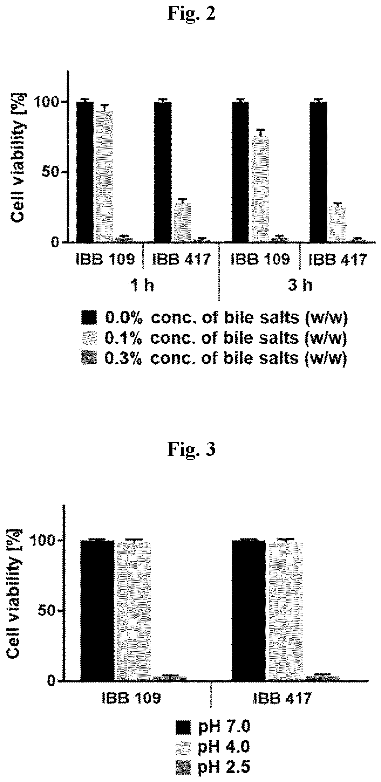

**Fig. 2****.** shows the results of the study of the survival *of L. lactis* IBB109 and *L. lactis* IBB417 strains in various concentrations of bile salts [0.1% (w/w) and 0.3% (w/w)] after incubation for 1.5 and 3 hours.
**Fig. 3****.** shows the results of the study of the survival *of L. lactis* IBB109 and *L. lactis* IBB417 strains under low pH (2.5 and 4) conditions in relation to the control pH (7) after a 2-hour incubation.

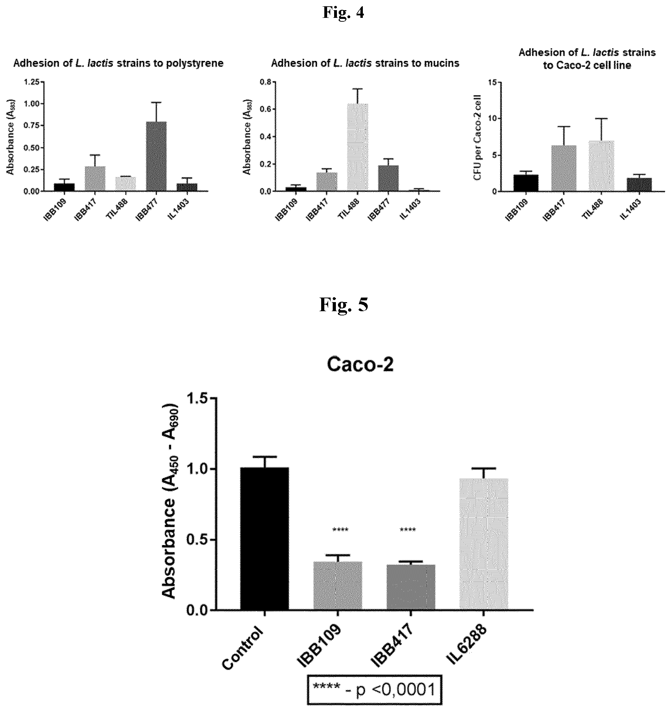

**Fig. 4****.** shows the results of the study of the adhesion capabilities *of L. lactis* IBB109 and *L. lactis* IBB417 strains to abiotic (polystyrene) and biotic (mucins and Caco-2 cells) factors in relation to the well-adherent strains (positive control: *L. lactis* IBB477 in the adhesion test to abiotic surfaces, *L. lactis* TIL488 in the mucin and Caco-2 line adhesion test).
**Fig. 5****.** shows the results of the study of the effect of live *L. lactis* IBB109 and *L. lactis* IBB417 strains on the level of inhibition of Caco-2 cell proliferation of in relation to the control strain *(L. lactis* IL6288) and the cell line not incubated with bacteria. Error bars represent the standard error. The results presented in the chart show statistically significant changes.

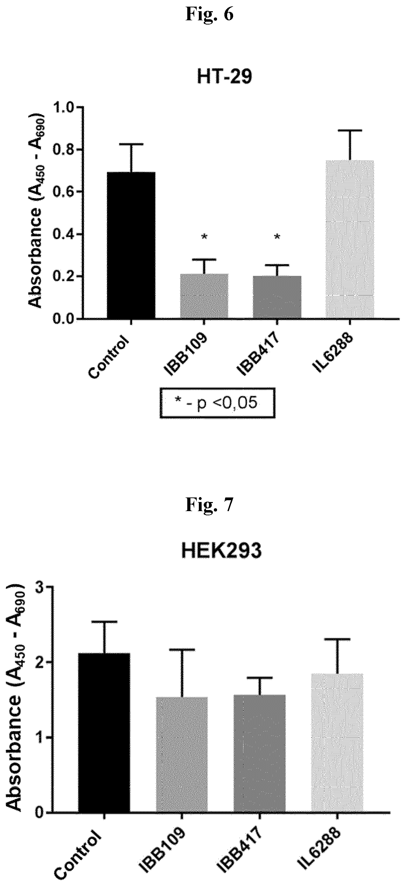

**Fig. 6****.** shows the results of the study of the effect of live *L. lactis* IBB109 and *L. lactis* IBB417 strains on the level of inhibition of HT-29 cell proliferation in relation to the control strain (*L*. *lactis* IL6288) and the cell line not incubated with bacteria. Error bars represent the standard error. The results presented in the chart show statistically significant changes.
**Fig. 7****.** shows the results of the study of the effect of live *L. lactis* IBB109 and *L. lactis* IBB417 strains on the level of inhibition of proliferation of non-cancerous, healthy HEK293 renal epithelial cells in relation to the control strain *(L. lactis* IL6288) and the cell line not incubated with bacteria. Error bars represent the standard error.

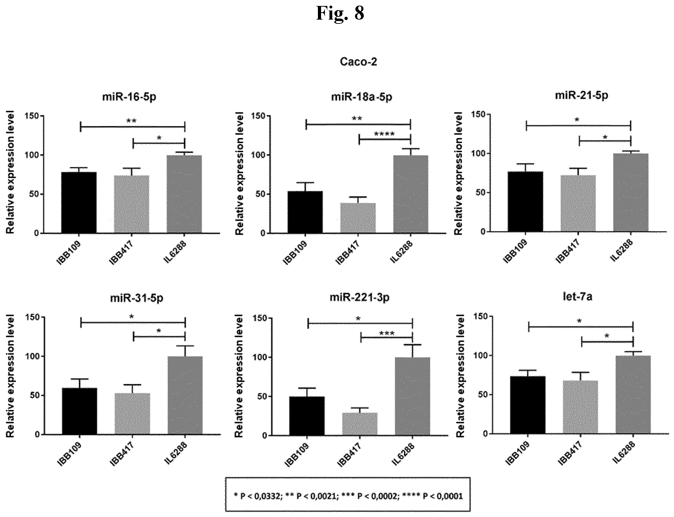

**Fig. 8****.** shows the results of the study of the expression of selected microRNAs in Caco-2 colon cancer cells under the influence *of L. lactis* IBB109 and *L. lactis* IBB417 strains and a control strain *(L. lactis* IL6288). The expression level was tested by real-time PCR (RT-qPCR). The results presented in the chart show statistically significant changes.

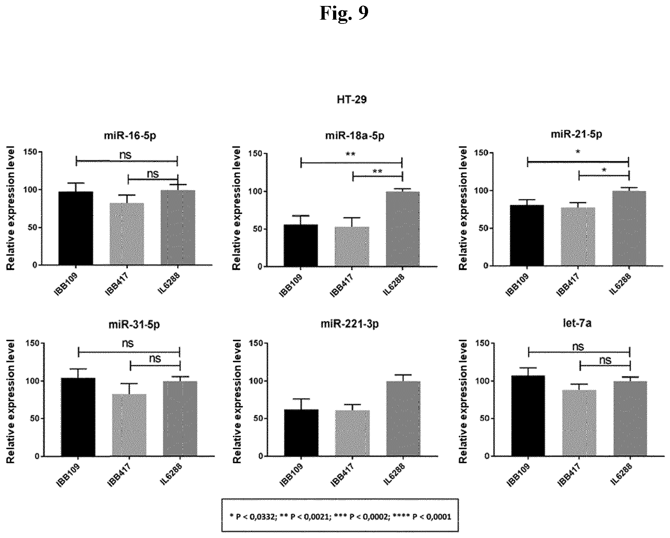

**Fig. 9****.** shows the results of the study of the expression of selected microRNAs in HT-29 colorectal cancer cells under the influence *of L. lactis* IBB109 and *L. lactis* IBB417 strains and a control strain *(L. lactis* IL6288). Expression levels were tested by real-time PCR (RT-qPCR). Error bars represent the standard error. Statistically significant results are marked accordingly in the diagram.

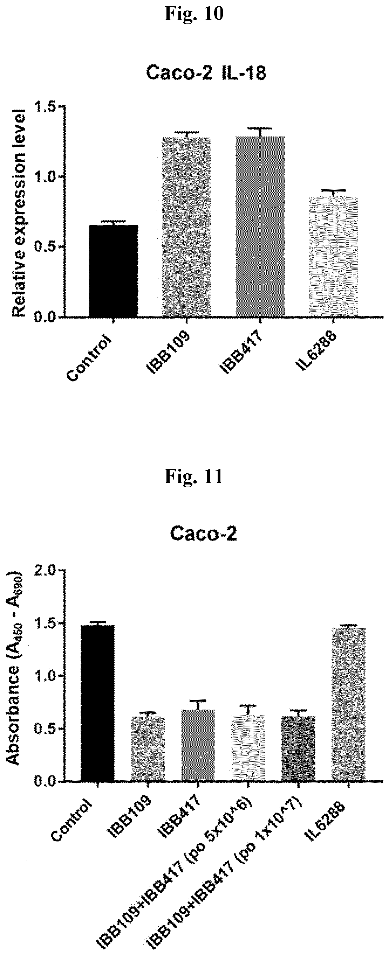

**Fig. 10****.** shows the results of the study of the expression of the interleukin 18 gene in the colorectal cancer Caco-2 cells under the influence *of L. lactis* IBB109 and *L. lactis* IBB417 strains and a control strain *(L. lactis* IL6288). Expression levels were tested by real-time PCR (RT-qPCR). Error bars represent the standard error.
**Fig. 11****.** shows the results of the study of the effect of live *L. lactis* IBB109 and *L. lactis* IBB417 strains separately and together on the colorectal cancer Caco-2 cells in relation to the control strain *(L. lactis* IL6288) and the cell line not incubated with bacteria. Error bars represent the standard error.

Publications cited in the description, and the references given therein, are in their entirety incorporated herein as references. The following examples illustrate the invention without limiting it in any way.

### EMBODIMENTS OF THE INVENTION

The following examples are provided merely to illustrate the invention and to explain particular aspects thereof, and not to limit it in any way, and are not to be interpreted as limiting the scope of the invention which is defined in the appended claims. In the following examples, unless it was otherwise indicated, standard materials and methods used in the field were used, or the manufacturers' instructions for specific materials and methods were followed.

### EXAMPLES

### Example 1. Selection of L. lactis IBB109 and IBB417 strains

The IBB PAN "COLIBB" collection of strains, including yeast, bacterial, bacteriophage strains and plasmids, includes several hundred strains of lactic acid bacteria isolated from natural sources, such as cow's milk, kefir, regional cheeses, regional sheep cheese "bryndza". For screening using the Caco-2 (ATCC HTB-37) cell line, 50 strains of *Lactococcus* bacteria were selected from the collection for which RAPD-DNA analysis of their genome was previously performed using primers B06: 5'-TGCTCTGCCC-3' (SEQ ID. NO. 15) and B08: 5'-GTCCACACGG-3' (SEQ ID. NO. 16). The results of the study showed a different pattern of signals suggesting genetic differences of individual strains, and thus probable differences in physiological properties. The selected strains, including IBB109 and IBB471 strains, were grown at 30°C, under aerobic or microaerophilic conditions, on solid or liquid M17 + 0.5% glucose (GM17) media and stored as stocks in 15% (v/v) glycerol at -80°C. The research described below was carried out on the selected set of 50 strains. Due to the special properties demonstrated for the two strains according to the invention, they were deposited on September 24, 2020 in the PCM (collection recognized for patent purposes) under deposit numbers: **B/00311** - *Lactococcus lactis* IBB 109 strain; **B/00312** - *Lactococcus lactis* IBB417 strain.

The *L. lactis* IBB109 strain was isolated from fresh cow's by seeding it on a solid GM17 medium. The *L. lactis* IBB417 strain was isolated from traditionally produced sheep cheese "bryndza" by inoculation on GM17 medium.

### Example 2. Effect of L. lactis IBB109 and L. lactis IBB417 strains on the proliferation of colorectal cancer cells

Fifty selected *Lactococcus* strains (see **Example 1**) were tested for their effect on cell proliferation of the Caco-2 colorectal cancer line.

For this purpose, the Caco-2 cell line was grown under the optimal conditions recommended by the ATCC, i.e. at 37°C, 5% CO₂, 95% humidity, in a medium (hereinafter called Caco-2 medium) consisting of MEM (Minimal Essential Medium; Gibco) with 10% fetal bovine serum, nonessential amino acid solution (NEAA) (1X), 1 mM sodium pyruvate, penicillin (100 U/mL) and streptomycin (100 µg/mL). Subsequently, the number of Caco-2 cells was determined in a Thoma chamber, diluted in the above-mentioned Caco-2 medium to obtain 10⁵ cells/mL and seeded on 96-well plate by adding 100 µl of cell suspension per well, and incubated for 24 h.

Bacterial cells of *Lactococcus* strains were prepared by culturing them in GM17 liquid medium (16 h, 30°C), and after centrifugation, by washing them twice with phosphate-buffered saline (pH 7.4) - PBS. Subsequently, bacterial suspension was prepared in the medium for Caco-2 (without the addition of antibiotics), corresponding to 10⁸ cfu/mL

Bacterial cells prepared in this way were applied to 96-well plates (100 µl per well) containing 24-hour grown Caco-2 cells, collecting the growth medium beforehand. The plates were incubated for 72 hours at 37°C, 5% CO₂, 95% humidity. The cell line viability was assessed by a colorimetric method using the commercially available Cell Proliferation Kit, BrdU (Roche) proliferation assay. The experiment was performed in 10 technical repeats. Caco-2 cells without bacteria served as control.

Out of 50 strains tested with this method, *L. lactis* IBB109 and IBB417 strains showed the strongest anti-proliferative activity against the Caco-2 cell line and were selected for further research. (**Fig. 1**).

### Example 3. Assessment of the biosafety level of L. lactis IBB109 and IBB417 strains

It is required that bacterial strains/preparations for use in the therapy/prevention of cancer development have documented biosafety properties. Hence, studies were conducted to assess the biological safety *of L. lactis* IBB109 and IBB417 strains in terms of their ability to induce the breakdown of red blood cells (hemolysis) and sensitivity to antibiotics listed in the document of the European Food Safety Authority (EFSA; EFSA Journal 2012;10(6):2740). The lack of hemolysis was confirmed by streak-plating the strains on a MacConkey medium enriched with 5% sheep blood. The plates were incubated for 48 hours at 37°C in an aerobic condition. The absence of clearance in the inoculated zone after 24 and 48 hours confirmed that both strains, IBB109 and IBB417 did not have hemolytic properties (Table 1). The evaluation of the resistance of the strains to antibiotics was carried out using the microdilution method in accordance with the ISO10932 standard against the control strain of *L. lactis* ATCC 19435. The obtained results indicate a similar level of sensitivity of both tested strains (Table 1), which was below or equal to the value of the minimum inhibitory concentration - MIC, for each of the 9 antibiotics. Due to the fact that the obtained MIC values were below the cut-off point given by EFSA, *L. lactis* IBB109 and *L. lactis* IBB417 strains were considered susceptible to the tested antibiotics.

**Table 1. Results of the studies on the resistance of the L. lactis IBB109 (B/00311) and L. lactis IBB417 (B/00312) strains to the selected antibiotics and hemolytic activity.**

| | Minimum inhibitory concentrations (MIC) (mg/L) | | | EFSA cut-off point (mg/L) |
|---|---|---|---|---|
| Antibiotic | IBB 109 | IBB417 | *Lactococcus lactis* ATCC 19435 EFSA (reference control strain) | |
| Gentamicin | 1 | 1-2 | 0.5-4 | 32 |
| Kanamycin | 8-16 | 8 | 2-8 | 64 |
| Streptomycin | 8 | 8-16 | 2-16 | 32 |
| Tetracycline | 0.5 | 0.25-0.5 | 0.5-2 | 4 |
| Erythromycin | 0.063 | 0.032-0.063 | 0.12-0.5 | 1 |
| Clindamycin | 0.032-0.063 | 0.032 | 0.25-0.5 | 1 |
| Chloramphenicol | 4 | 4 | 4-16 | 8 |
| Ampicillin | 0.25-0.5 | 0.5 | 0.5-1 | 2 |
| Vancomycin | 0.25 | 0.25 | 0.25-1 | 4 |
| | | | | |
| | IBB109 | IBB417 | | |
| Hemolysis | | | | |

The lack of antibiotic resistance genes was also confirmed by sequencing the genomic DNA of *L. lactis* IBB 109 and *L. lactis* IBB 17 strains carried out by the Laboratory of DNA Sequencing and Oligonucleotide Synthesis of IBB PAN (Illumina, MiSeq oraz MinION, NanoPore). The analysis of complete DNA sequences (chromosomal and plasmid) showed that both strains do not carry any resistance genes, which is extremely important for the plans in using them in the food industry, and for the safe use in dietary supplements, nutraceuticals, and pharmaceutical preparations and compositions.

### Example 4. Assessment of probiotic features of unique L. lactis IBB109 and IBB417 strains

The study of the strains in terms of probiotic properties included testing the resistance of bacterial cells to bile salts and low pH, as well as assessing their adhesion to abiotic (polystyrene) and biotic factors (mucins and Caco-2 cells). Bacteria for testing were obtained by culturing them overnight in GM17 liquid medium (30°C, aerobic conditions), followed by centrifugation and suspension of the cell pellets in saline solution to obtain a final optical density (OD₆₀₀) of 1.

Resistance to bile salts was tested by adding 1 ml of bacterial suspension (at OD₆₀₀ 1) to 9 ml of bile salts solutions (Sigma cat. No. B8756) at a final concentration of 0.1% (w/w) or 0.3% (w/w) and incubation for 1.5 or 3 hours at 37°C. After incubation, serial dilutions of bacterial cells were plated on GM17 solid medium and after 48-hour cultivation at 30°C the number of the obtained colonies were counted to determine the number of colony forming units per 1 mL (CFU/mL) (**Fig. 2**). The obtained results indicated a better survival and thus a higher resistance of the IBB109 strain to 0.1% bile salt concentration in relation to IBB417; after both 1.5 and 3 hours of incubation under the above-mentioned conditions, no decrease in the number of cells of the IBB109 strain was observed. The higher concentration of bile salts (0.3%) decreased the survival of both strains after the 1.5-hour incubation. The resistance of the strains to low pH was tested by adding 1 ml of bacterial suspension (at OD₆₀₀ 1) to 9 ml of saline at pH 4 or pH 2.5 and incubation for 2 hours at 37°C. After incubation, serial dilutions of bacterial cells were plated on GM17 solid medium and after 48-hour cultivation at 30°C the number of grown colonies were counted to determine the CFU/mL, in comparison to same strains incubated in a saline solution at pH 7. The obtained results show that the 2-hour incubation at pH 4.0 did not affect the viability of both strains, while pH 2.5 turned out to be lethal for the cells of the tested bacteria (**Fig. 3**).

The ability of bacteria to adhere to polystyrene or mucins was tested according to known methodology (Radziwill-Bieńkowska et al. 2016) on 96-well plates, uncoated or coated with pig stomach mucins, respectively. For this purpose, 100 µl of the bacterial suspensions (at OD₆₀₀ 1) were applied per well and incubated for 3 hours at 30°C under aerobic conditions. After this time, the plates were washed three times with PBS buffer, stained for 10 minutes with crystal violet, washed three times with deionized water, flooded with 96% ethanol and allowed to evaporate for an hour, and measured at 583 nm using a spectrophotometer. Strains recognized as well-adhering under the tested conditions served as controls - *L. lactis* IBB477 (for adhesion test to abiotic surfaces) (Radziwill-Bieńkowska et al. 2016) and *L. lactis* TIL488 (for mucin adhesion tests) (Le et al. 2013).

Adhesion to mammalian cells was performed on differentiated Caco-2 cells. For this, cells were passaged in Caco-2 medium in 12-well plates for 14 days at 37°C, 5% CO₂, 95% humidity, changing the culture medium every 3 days. The bacteria were grown in GM17 liquid medium for 16 hours, and then the cells were centrifuged, washed twice with cold PBS buffer and resuspended in Caco-2 medium without antibiotics (streptomycin and ampicillin) in an amount corresponding to 10 CFU of bacteria per one Caco-2 cell. Bacteria prepared in this way were added to each well containing differentiated (2-week-old) Caco-2 cells, collecting beforehand the growth medium, and incubated for 3 hours under conditions optimal for the Caco-2 cell line. Differentiated Caco-2 cells incubated with the well-adherent *L. lactis* TIL448 strain served as a control (Le et al. 2013). Subsequently, the tissue cultures were washed with PBS buffer, the supernatant was collected and the unattached bacterial cells were counted. To determine the number of adhered bacterial cells, Caco-2 cells were gently lysed in PBS buffer supplemented with TRITON X100 at a final concentration of 0.1%. Subsequently, serial dilutions of bacterial cells were plated on GM17 solid medium, and after 48-hour cultivation at 30°C, the number of obtained colonies was counted to determine the CFU/mL. Based on the obtained results, the IBB417 strain showed a higher level of adhesion to polystyrene and mucins, compared to the control strain (non-adherent *L. lactis* IL1403 strain), but still lower lthan the control strains considered to adhere well under these conditions (strains *L. lactis* IBB477 and TIL488, respectively) (**Fig. 4**). In contrast, in studies using the Caco-2 cell line, the IBB417 strain showed a comparable level of adhesion as the positive control *(L. lactis* TIL488 strain) (**Fig. 4**). This is a beneficial feature, allowing for longer survival/presence of this strain in the digestive system, and thus enabling a prolonged impact on the host organism. On the other hand, the IBB109 strain did not show equally strong adhesive properties under the tested conditions.

### Example 5. Colorectal cancer cell proliferation inhibition test using L. lactis IBB109 and IBB417 strains

The effect of IBB109 and IBB417 strains on the inhibition level of cell proliferation of two colorectal cancer lines (Caco-2, HT-29) and of a healthy, non-cancerous renal epithelial cell line (HEK-293) was tested in comparison to the untreated respective cell lines and respective cell lines incubated with the control strain *L. lactis* IL6288 (Bolotin et al. 2019) shown not to affect colorectal cancer cells Caco-2 and HT-29 and serving as a negative control strain. The proliferation inhibition test was performed using the Cell Proliferation ELISA kit, BrdU (Roche). Statistical analyzes were performed using Shapiro-Wilk tests, multivariate analysis of variance (ANOVA). The effect of the strains on the proliferation of colorectal cancer cells was assessed by a 72-hour incubation of Caco-2, HT-29 and HEK-293 cells with live bacterial cells at stationary phase. Cultures of cell lines and bacteria were carried out in the same way as in **Example 2**. The *L. lactis* IBB109 and *L. lactis* IBB417 strains showed a very strong inhibition of proliferation of colorectal adenocarcinoma cells of the Caco-2 and HT-29 cell lines *in vitro* (**Fig. 5****,** **Fig. 6**). Such effect was not observed for the healthy, non-cancer cell line derived from the renal epithelium HEK-293 (**Fig. 7**) and any differences were not statistically significant. This indicates that *L. lactis* IBB109 and *L. lactis* IBB417 strains will not affect the proliferation of normal epithelial cells, which is extremely important for use in the food industry and in applications of the strains as dietary supplements, nutraceuticals and in pharmaceutical preparations.

### Example 6. The influence of L. lactis IBB109 and IBB417 on the expression of miRNA involved in the proliferation of colorectal cancer cells

The expression levels of six microRNA molecules (miR-16-5p, miR-18a-5p, miR-21-5p, miR-31-5p, miR-221-3p and let-7a), selected based on the literature data as related to the regulation of the proliferation process in colorectal cancer cells, were tested. For this purpose, Caco-2 and HT-29 cell lines were grown analogously as in **Example 2,** and then total RNA was isolated from them and the expression level of individual miRNAs was determined by RT-qPCR using specific primer pairs (**Table 2**). The U48 gene was used as the reference gene.

**Table 2. Primer pair sequences (R-reverse, F-forward) were used to analyze the levels of selected miRNAs.**

| **SEQ ID. NO.** | **miRNA** | **Primer name** | **Primer sequence 5' - 3'** |
|---|---|---|---|
| 1 | **let-7a** | let-7a_F | gcagtgaggtagtaggttg |
| 2 | | let-7a_R | gtccagtttttttttttttttaactatac |
| 3 | **miR-21-5p** | miR_21_F | gcagtagcttatcagactgatg |
| 4 | | miR_21_R | ggtccagtttttttttttttttcaac |
| 5 | **U48** | U48_F | accccaggtaactcttgagtgtgtcgctgatgccatcac cgcagcgctct |
| 6 | | U48_R | gtccagtttttttttttttttggtc |
| 7 | **miR-221-3p** | miR_221_F | gcagagctacattgtctgct |
| 8 | | miR_221_R | gagtttttttttttttttgaaaccca |
| 9 | **miR-18a-5p** | miR_18a_F | gtaaggtgcatctagtgcag |
| 10 | | miR_18a_R | ggtccagtttttttttttttttctatc |
| 11 | **miR-31-5p** | miR_31_F | gcagaggcaagatgctg, |
| 12 | | miR_31_R | gtccagtttttttttttttttagctatg |
| 13 | **miR-16-5p** | miR_16_F | cgcagtagcagcacgta |
| 14 | | miR_16_R | cagtttttttttttttttcgccaa |

The results for Caco-2 cells after incubation with the *L. lactis* IBB109 and IBB417 strains show a statistically significant reduction in the expression level of all tested miRNAs compared to the levels of miRNAs in cells incubated with the control strain *(L. lactis* IL6288), showing no anti-proliferative properties against Caco-2 cells (**Fig. 8**). The results obtained for HT-29 cells after incubation with *L. lactis* IBB109 and IBB417 strains show for four out of six analyzed miRNA molecules, i.e. miR-16-5p, miR-18a-5p, miR-21-5p, miR-221-3p, a reduction in the expression level in relation to the miRNA level in cells incubated analogously with the same control strain (**Fig. 9**). Unexpectedly, it was found that *L. lactis* IBB109 and *L. lactis* IBB417 strains, according to the invention, are capable of modulating the expression level of miRNA molecules in neoplastic cell lines, including colorectal cancer cell lines.

### Example 7. Effect of L. lactis IBB109 and IBB417 strains on the level of IL-18 in mammalian cells

The effect of strains on the level of IL-18 production in colorectal cancer cells was assessed by incubating 24-hour cultures of Caco-2 cells with live bacterial cells from the stationary phase. Bacterial and cell line cultures were carried out in the same way as in **Example 2.** Total RNA was then isolated from the Caco-2 cell line and the expression level of IL-18 was determined by RT-qPCR using specific primers: IL-18F (SEQ ID. NO. 17; 5'-atcgcttcctctcgcaacaa-3'), IL-18R (SEQ ID. NO. 18; 5'-cttctactggttcagcagccatct-3'). The results of the analyzes showed that the presence *of L. lactis* IBB109 and *L. lactis* IBB417 strains increases the production of interleukin 18 in Caco-2 cells compared to the control strain *(L. lactis* IL6288) (**Fig. 10**). Hence, the use of these strains may contribute to the stimulation of the immune system and earlier recognition of cancer cells by the host organism and thus prevent the development of a neoplastic disease.

### Example 8. Incubation of colorectal adenocarcinoma cell lines with L. lactis IBB109 and IBB417 strains

The simultaneous effect of both strains on the proliferation of colorectal cancer cells was assessed by a 72-hour incubation of Caco-2 cell with live bacterial cells from the stationary phase of both strains (IBB109 and IBB417). Bacterial and cell line cultures were carried out in the same way as in **Example 2.** The *L. lactis* IBB109 and IBB417 strains were incubated with the cell line in two concentration variants: 5×10⁶ CFU or 10⁷ CFU of each strain per well. The obtained results indicate that with the simultaneous administration of the IBB109 and IBB417 strains, regardless of the tested concentration, the inhibition level of Caco-2 colorectal adenocarcinoma cells proliferation was similar as when each strain assayed separately (**Fig. 11**). This means that the strains do not exert mutual antagonistic effects and can be administered simultaneously. Due to the different probiotic properties of the two strains, it cannot be ruled out that their simultaneous administration may have a positive effect and lead to a synergistic enhancement of their activity. The possibility of simultaneous administration of both strains is advantageous due to the different/complementary probiotic properties of both strains.

### Summary and conclusions:

The novel bacterial strains, *L. lactis* IBB109 and IBB417, according to the invention were isolated from natural sources (cow's milk, regional sheep cheese "bryndza") and, based on the screening tests, characterized as strains of the genus *Lactococcus* with a different genetic profile (**Example *1***)*. Lactococcus lactis* strains selected from various natural sources were tested for their effect on the proliferation of Caco-2 colorectal cancer cells (**Example 2**). Only two of the multiple strains of the genus *Lactococcus* that were analyzed, i.e. *Lactococcus lactis* IBB109, deposited as B/00311, and *Lactococcus lactis* IBB417, deposited as B/00312, showed a strong anti-proliferative effect against the Caco-2 cell line (**Fig. 1**). Further studies showed a significantly strong effect of the *L. lactis* IBB109 and IBB417 strains on the inhibition level of proliferation of various colorectal cancer lines (**Caco-2, HT-29)** and the lack of a similar effect on healthy, non-cancerous epithelial cells, i.e. renal epithelial cells (**HEK-293**), and in comparison to untreated respective cell lines and respective cell lines incubated with another *L. lactis* strain as a negative control (**Example 5**). The results indicate a statistically significant inhibition of the proliferation of Caco-2 and HT29 colorectal cancer cells under the influence of the tested *L. lactis* IBB109 and IBB417 strains compared to the control, which shows that the observed changes are strain-dependent (**Fig. 5****,** **Fig. 6**). At the same time, *L. lactis* IBB109 and IBB417 strains did not show an anti-proliferative effect on the healthy, non-cancerous epithelial cell line derived from renal epithelium, HEK-293 (**Fig. 7**). It has been shown that it is possible to incubate the Caco-2 cell line with a bacterial culture that is a mixture of both *L. lactis* IBB109 and IBB417 strains, which also leads to a strong inhibition of proliferation (**Example 8,** **Fig. 11**). At the same time, the use of both strains will be beneficial for the obtained therapeutic effects due to the different probiotic properties and genetic features, suggesting potential differences in the mechanisms of action of *L. lactis* IBB109 and IBB417 (**Example 4**). Thus, the administration of both strains simultaneously or sequentially will possibly enhance the preventive and/or therapeutic effect of these strains on intestinal cancers, particularly cancers of the large intestine.

Bacterial cultures of the studied strains were also analyzed in terms of their influence on the expression level of selected microRNA molecules, which are regulators of the neoplastic process (**Example 6**). It was shown that the incubation of bacterial cultures of *L. lactis* IBB 109 and IBB417 strains (according to the invention) with the Caco-2 cell line resulted in a statistically significant reduction in the levels of the tested microRNA molecules, but no such changes were observed in the presence of the control strain, lacking the anti-proliferative effect against Caco-2 (**Fig. 8**). In the case of the HT-29 cell line, incubation *of L. lactis* IBB109 and IBB417 strains (according to the invention) induced changes in the expression level of selected microRNAs while no changes were observed in the presence of the control strain (**Fig. 9**). It has been shown that *L*. *lactis* IBB 109 and IBB417 strains according to the invention affect the regulation of the expression of microRNA molecules (oncomirs) that are associated with cancer development, including colorectal cancer. Both *L. lactis* IBB 109 and IBB417 strains, according to the invention, can therefore be used in the treatment and prevention of intestinal cancer, especially colorectal cancer.

The intestinal epithelial cells naturally produce interleukin 18 (IL-18) at a approximately constant level. In colorectal cancer cells there is a decrease in IL-18 level or its complete absence. This phenomena is postulated to be a form of escape of cancer cells from the host's immune system. A positive effect of bacterial cultures *of L. lactis* IBB109 and IBB417 strains according to the invention was demonstrated in respect to increase of cytokine IL-18 levels in the tested tumor cell lines (**Example 7**). Incubation of Caco-2 cell lines with *L. lactis* IBB 109 and IBB417 strain cells according to the invention has been shown to increase the level of pro-inflammatory IL-18, thus initiating an immune response (**Fig. 10**). The IL-18 stimulatory effect was not observed in case of the control strain, which was shown no to affect proliferation of the tested tumor lines. It is required that bacterial strains/preparations for use in therapy/prevention of cancer development have documented biosafety properties (no resistance to antibiotics in accordance with EFSA and the ISO10932 standard and no hemolytic properties). Both the *L. lactis* IBB109 and IBB417 strains, according to the invention, meet the biosafety requirements and can be used in preparations administered to humans, for example in the form of dietary supplements, nutraceuticals, functional food enriched with these strains or medicinal pharmaceutical compositions.

Bacterial strains to be used as bacterial preparations should exhibit a number of features essential for their survival and ability to persist in the digestive tract of the host organism. The key properties in this regard include: survival at low pH and in the presence of bile salts as well as the ability to adhere to epithelial cells. The *L. lactis* IBB 109 and IBB417 strains according to the invention were tested for the above-mentioned parameters (**Example 4**). The IBB417 strain showed adhesion to abiotic surfaces and mucins at a level higher than the control non-adherent strain and good adhesion to biotic surfaces (mammalian cells), especially to the cells of the colorectal cancer line Caco-2 - comparable to the adhesion of the recognized adhesive control strain (Fig. 4). The IBB 109 strain did not show equally strong adhesive properties under the tested conditions.

In conclusion, in the conducted studies it was shown that both strains are characterized by high resistance to low pH, the IBB 109 strain is less sensitive to bile salts compared to the IBB417 strain, while the IBB417 strain has better adhesive properties. It has been demonstrated that IBB109 and IBB417 strains can be co-administered, they do not show mutual antagonistic effects, and their simultaneous administration, due to different probiotic and genetic characteristics of both strains, can have a positive effect, leading to a potential enhancement of their effects.

The *L. lactis* IBB 109 and IBB417 strains according to the invention, when administered separately or together, exhibit anti-proliferative properties against colorectal cancer cell lines *in vitro,* what may find use in the prevention and treatment of neoplastic conditions, especially intestinal cancers, particularly colorectal cancers. The strains can therefore be a component of pharmaceutical compositions, dietary supplements, nutraceuticals and functional foods with strengthening, preventive and therapeutic properties against cancers, and particularly - intestinal cancers.

### LITERATURE

**(1)** Markowiak P, Slizewska K. Effects of probiotics, prebiotics, and synbiotics on human health. Nutrients. 2017 Sep 15; 9(9):1021.
**(2)** Liu Z, Qin H, Yang Z, Xia Y, Liu W, Yang J, Jiang Y, Zhang H, Yang Z, Wang Y, Zheng Q. Randomised clinical trial: the effects of perioperative probiotic treatment on barrier function and post-operative infectious complications in colorectal cancer surgery - a double-blind study. Aliment Pharmacol Ther. 2011 Jan; 33(1):50-63.
**(3)** Banna GL, Torino F, Marletta F, Santagati M, Salemi R, Cannarozzo E, Falzone L, Ferraù F, Libra M. Lactobacillus rhamnosus GG: An overview to explore the rationale of its use in cancer. Front Pharmacol. 2017 Sep 1; 8:603.
**(4)** Brenner H, Kloor M, Pox CP. Colorectal cancer. Lancet, 2014 Apr 26; 383(9927):1490-1502.
**(5)** Zhao H, Zhao C, Dong Y, Zhang M, Wang Y, Li F, Li X, McClain C, Yang S, Feng W. Inhibition of miR122a by Lactobacillus rhamnosus GG culture supernatant increases intestinal occludin expression and protects mice from alcoholic liver disease. Toxicol Lett. 2015 May 5; 234(3):194-200.
**(6)** Zhou W, Yuan Y, Li J, Yuan WM, Huang LG, Zheng SW. Effect of Bifidobacterium on the mRNA expression levels of TRAF6, GSK-3β, and microRNA-146a in LPS-stimulated rat intestinal epithelial cells. Genet Mol Res. 2015 Aug 21; 14(3):10050-10056.
**(7)** Deepak V, Ramachandran S, Balahmar RM, Pandian SR, Sivasubramaniam SD, Nellaiah H, Sundar K. In vitro evaluation of anticancer properties of exopolysaccharides from Lactobacillus acidophilus in colon cancer cell lines. In Vitro Cell Dev BiolAnim. 2016 Feb; 52(2):163-73.
**(8)** Wasilewska E, Zlotkowska D, Pijagin ME. The role of intestinal microflora and probiotic bacteria in prophylactic and development of colorectal cancer. Postepy Hig. Med. Dosw. 2013 Aug 9; 67:837-847.
**(9)** Collado MC, Isolauri E, Salminen S, Sanz Y. The impact of probiotic on gut health. Curr DrugMetab. 2009 Jan; 10(1):68-78.
**(10)** Mego M, Holec V, Drgona L, Hainova K, Ciernikova S, Zajac V. Probiotic bacteria in cancer patients undergoing chemotherapy and radiation therapy. Complement Ther Med. 2013 Dec; 21(6):712-723.
**(11)** Górska A, Przystupski D, Niemczura MJ, Kulbacka J. Probiotic Bacteria: A Promising Tool in Cancer Prevention and Therapy. Curr Microbiol. 2019 Aug;76(8):939-949.
**(12)** Jacouton E, Chain F, Sokol H, Langella P, Bermúdez-Humarán LG. Probiotic strain Lactobacillus casei BL23 prevents colitis-associated colorectal cancer. Front Immunol. 2017 Nov 17; 8:1553.
**(13)** Zhong L, Zhang X, Covasa M. Emerging roles of lactic acid bacteria in protection against colorectal cancer. World J Gastroenterol. 2014 Jun 28; 20(24):7878-7886.
**(14)** Yu AQ, Li L. The potential role of probiotics in cancer prevention and treatment. Nutr Cancer. 2016 May-Jun; 68(4):535-44.
**(15)** Yamane T, Sakamoto T, Nakagaki T, Nakano Y. Lactic acid bacteria from kefir increase cytotoxicity of natural killer cells to tumor cells. Foods. 2018 Mar 27; 7(4):48.
**(16)** Wu WK, Law PT, Lee CW, Cho CH, Fan D, Wu K, Yu J, Sung JJ. MicroRNA in colorectal cancer: from benchtop to bedside. Carcinogenesis. 2011 Mar; 32(3):247-53.
**(17)** Kreuzer-Redmer S, Bekurtz JC, Arends D, Bortfeldt R, Kutz-Lohroff B, Sharbati S, Einspanier R, Brockmann GA. Feeding of Enterococcus faecium NCIMB 10415 leads to intestinal miRNA-423-5p-induced regulation of immune-relevant genes. Appl Environ Microbiol. 2016 Apr 4; 82(8):2263-2269.
**(18)** Heydari Z, Rahaie M, Alizadeh AM, Agah S, Khalighfard S, Bahmani S. Effects of Lactobacillus acidophilus and Bifidobacterium bifidum probiotics on the expression of microRNAs 135b, 26b, 18a and 155, and their involving genes in mice colon cancer. Probiotics Antimicrob Proteins. 2019 Dec; 11(4):1155-1162.
**(19)** Le DT, Tran TL, Duviau MP, Meyrand M, Guérardel Y, Castelain M, Loubière P, Chapot-Chartier MP, Dague E, Mercier-Bonin M. Unraveling the role of surface mucus-binding protein and pili in muco-adhesion of Lactococcus lactis. PLoS One. 2013 Nov 18; 8(11):e79850.
**(20)** Radziwill-Bieńkowska JM, Le DT, Szczesny P, Duviau MP, Aleksandrzak-Piekarczyk T, Loubière P, Mercier-Bonin M, Bardowski JK, Kowalczyk M. Adhesion of the genome-sequenced Lactococcus lactis subsp. cremoris IBB477 strain is mediated by specific molecular determinants. Appl Microbiol Biotechnol. 2016 Nov; 100(22):9605-9617.
**(21)** Skrzydlo-Radomanska B, Wronecki J. Czy mikrobiot 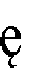 jelitow 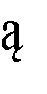 można skutecznie modyfikować? *Gastroenterologia Kliniczna,* 2018, 10(4), 123-134.
**(22)** Olszewska M, 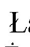 aniewska-Trokenheim 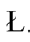 . Odpowiedź bakterii fermentacji mlekowej na stres - Stadium Vbnc. *Żywność. Nauka. Technologia. Jakość*, 2013, 5 (90), 15 - 28.
**(23)** Takanashi S, Miura A, Abe K, Uchida J, Itoi S, Sugita H. Variations in bile tolerance among Lactococcus lactis strains derived from different sources. FoliaMicrobiol. (Praha). 2014 Jul; 59(4): 289-93.
**(24)** Jatmiko YD, Howarth GS, Barton MD. Assessment of probiotic properties of lactic acid bacteria isolated from Indonesian naturally fermented milk. AIP Conference Proceedings 1908, 050008 (2017); https://doi.org/10.1063/1.5012732.
**(25)** Guidance on the assessment of bacterial susceptibility to antimicrobials of human and veterinary importance. EFSA Journal, 2012; 10(6): 2740
**(26)** Bolotin A, Aucouturier A, Sorokin A, Bidnenko E.. Genomic sequence of the prophagefree Lactococcus lactis strain IL6288. Microbiol Resour Announc. 2019, 8:e01545-18. https://doi.org/10.1128/MRA.01545-18.

### Sequence Listing

The list of sequences **SEQ ID. NO. 1-18** is presented in the description and in the electronic version of the sequence list sent with the application.

## Claims

1. A novel bacterial strain *Lactococcus lactis* IBB109 deposited in PCM under deposit no. B/00311.

2. A novel bacterial strain *Lactococcus lactis* IBB417 deposited in PCM under deposit no. B/00312.

3. A composition containing a novel bacterial strain *Lactococcus lactis* IBB109 deposited in PCM under deposit no. B/00311 and a novel bacterial strain *Lactococcus lactis* IBB417 deposited in PCM under deposit no. B/00312.

4. The composition according to claim 3, **characterized in that** the composition is a pharmaceutical composition further comprising a pharmaceutically acceptable carrier.

5. The composition according to claims 3-4, **characterized in that** it is in the form of a liquid, solid, powder, tablet, capsule intended for oral administration.

6. A pharmaceutical composition comprising bacterial strain *Lactococcus lactis* IBB109 as defined in claim 1 and/or bacterial strain *Lactococcus lactis* IBB417 as defined in claim 2 and/or a composition as defined in claim 3 and a pharmaceutically acceptable carrier for use as a medicament.

7. The pharmaceutical composition according to claim 6, **characterized in that** it is for use as a medicament in the prevention and/or treatment of intestinal cancer, preferably in the prevention and/or treatment of colorectal cancer.

8. The pharmaceutical composition according to claims 6-7, **characterized in that** it is in the form of a liquid, solid, powder, tablet, capsule intended for oral administration.

9. A dietary supplement comprising the bacterial strain *Lactococcus lactis* IBB109 as defined in claim 1 and/or a strain of *Lactococcus lactis* IBB417 bacteria as defined in claim 2 and/or a composition as defined in claim 3, wherein the dietary supplement is preferably in the form of a liquid, solid, powder, tablet, capsule intended for oral administration.

10. A probiotic preparation comprising the bacterial strain *Lactococcus lactis* IBB109 as defined in claim 1 and/or the bacterial strain *Lactococcus lactis* IBB417 as defined in claim 2 and/or a composition as defined in claim 3, wherein the probiotic preparation preferably further comprises prebiotic substances, preferably selected from oligosaccharides, polysaccharides, fructooligosaccharides, lactulose, inulin, resistant starch, cellulose, hemicellulose and pectins, wherein, preferably the preparation further comprises postbiotics, preferably postbiotics are selected from short chain fatty acids, enzymes, lipopolysaccharides, teichoic acids, vitamins, butyric acid, acetate, propionate, muramil dipeptide, indol, teichic acid, lactocepins.

11. A probiotic preparation according to claim 10, **characterized in that** it is in the form of a liquid, solid, powder, tablet, capsule intended for oral administration.

12. A bacterial preparation containing the bacterial strain *Lactococcus lactis* IBB109 as defined in claim 1 and/or the bacterial strain *Lactococcus lactis* IBB417 as defined in claim 2 and/or a composition as defined in claim 3 for use as an active ingredient in a probiotic, therapeutic preparation, functional food, dietary supplement, for use as an active ingredient of a medicament intended for the prevention and/or treatment of an intestinal cancer, preferably a colorectal cancer.

13. The bacterial preparation according to claim 12, **characterized in that** it further comprises prebiotic substances, preferably selected from oligosaccharides, polysaccharides, fructooligosaccharides, lactulose, inulin, resistant starch, cellulose, hemicellulose and pectins, wherein, preferably, the preparation further comprises postbiotics, preferably postbiotics being selected from short-chain fatty acids, enzymes, lipopolysaccharides, teichoic acids, vitamins, butyric acid, acetate, propionate, muramil dipeptide, indol, teichic acid, lactocepins.

14. The use of the bacterial strain *Lactococcus lactis* IBB109 as defined in claim 1 and/or the bacterial strain *Lactococcus lactis* IBB417 as defined in claim 2 and/or a composition as defined in claim 3 as a functional additive in a functional food, a functional drink additive, as an active ingredient in a probiotic preparation, a bacterial preparation, a dietary supplement, a pharmaceutical composition.

15. The use of the bacterial strain *Lactococcus lactis* IBB109 as defined in claim 1 and/or the bacterial strain *Lactococcus lactis* IBB417 as defined in claim 2 and/or a composition as defined in claim 3 for inducing a level of the cytokine IL-18 in intestinal cancer cells, preferably in colorectal cancer cells.


SEQUENCE LISTING

<110>  IBB

<120>  Nowe szczepy  Lactococcus lactis, kompozycje je obejmuj¹ce oraz
       ich zastosowanie w zapobieganiu i leczeniu nowotworu raka jelita

<130>  PPL1120AGR

<160>  18

<170>  PatentIn version 3.5

<210>  1
<211>  19
<212>  DNA
<213>  Homo sapiens

<400>  1
gcagtgaggt agtaggttg                                                    19


<210>  2
<211>  30
<212>  DNA
<213>  Homo sapiens

<400>  2
ggtccagttt tttttttttt ttaactatac                                        30


<210>  3
<211>  22
<212>  DNA
<213>  Homo sapiens

<400>  3
gcagtagctt atcagactga tg                                                22


<210>  4
<211>  26
<212>  DNA
<213>  Homo sapiens

<400>  4
ggtccagttt tttttttttt ttcaac                                            26


<210>  5
<211>  50
<212>  DNA
<213>  Homo sapiens

<400>  5
accccaggta actcttgagt gtgtcgctga tgccatcacc gcagcgctct                  50


<210>  6
<211>  25
<212>  DNA
<213>  Homo sapiens

<400>  6
gtccagtttt tttttttttt tggtc                                              25


<210>  7
<211>  20
<212>  DNA
<213>  Homo sapiens

<400>  7
gcagagctac attgtctgct                                                    20


<210>  8
<211>  26
<212>  DNA
<213>  Homo sapiens

<400>  8
   cagttttttt ttttttttga aaccca                                              26


<210>  9
<211>  20
<212>  DNA
<213>  Homo sapiens

<400>  9
gtaaggtgca tctagtgcag                                                    20


<210>  10
<211>  27
<212>  DNA
<213>  Homo sapiens

<400>  10
ggtccagttt tttttttttt ttctatc                                            27


<210>  11
<211>  17
<212>  DNA
<213>  Homo sapiens

<400>  11
gcagaggcaa gatgctg                                                       17


<210>  12
<211>  28
<212>  DNA
<213>  Homo sapiens

<400>  12
gtccagtttt tttttttttt tagctatg                                           28


<210>  13
<211>  17
<212>  DNA
<213>  Homo sapiens

<400>  13
gcagaggcaa gatgctg                                                    17


<210>  14
<211>  24
<212>  DNA
<213>  Homo sapiens

<400>  14
cagttttttt tttttttcg ccaa                                             24


<210>  15
<211>  10
<212>  DNA
<213>  Homo sapiens

<400>  15
tgctctgccc                                                            10


<210>  16
<211>  10
<212>  DNA
<213>  Homo sapiens

<400>  16
gtccacacgg                                                            10


<210>  17
<211>  20
<212>  DNA
<213>  Homo sapiens

<400>  17
atcgcttcct ctcgcaacaa                                                 20


<210>  18
<211>  24
<212>  DNA
<213>  Homo sapiens

<400>  18
cttctactgg ttcagcagcc atct                                            24